# EUROPEAN PATENT SPECIFICATION

(11) **EP 4 424 660 B1**
(45) Date of publication and mention of the grant of the patent: **25.02.2026**
(21) Application number: 23892782.6
(22) Date of filing: 25.05.2023
(51) Int. Cl.: C07C 29/76, B01D 1/00, B01D 1/26, B01D 9/02, C07C 29/78, C07C 31/18

(54) **PREPARATION SYSTEM AND METHOD FOR XYLITOL CRYSTAL**
SYSTEM UND VERFAHREN ZUR HERSTELLUNG VON XYLITOLKRISTALL
SYSTÈME ET PROCÉDÉ DE PRÉPARATION DE CRISTAL DE XYLITOL

(30) Priority: 07.12.2022 CN 202211567511
(43) Date of publication of application: 04.09.2024
(73) Proprietor: Zhejiang Huakang Pharmaceutical Co., Ltd., Quzhou, Zhejiang 324302 (CN)
(72) Inventor: WU, Qiang, Quzhou, Zhejiang 324302 (CN); LI, Mian, Santa Clara, CA (US); YANG, Wulong, Quzhou, Zhejiang 324302 (CN); WAN, Fuan, Quzhou, Zhejiang 324302 (CN); XU, Weidong, Quzhou, Zhejiang 324302 (CN); QIN, Shufang, Quzhou, Zhejiang 324302 (CN); YUE, Hang, Quzhou, Zhejiang 324302 (CN); WANG, Weijia, Quzhou, Zhejiang 324302 (CN)
(74) Representative: Sun, Yiming
(86) International application number: PCT/CN2023/096364
(87) International publication number: WO 2024/119731

(56) References cited:
- CN-A- 101 628 853
- CN-A- 108 837 550
- CN-A- 111 777 493
- CN-A- 114 163 306
- CN-A- 114 478 191
- CN-A- 115 784 843
- CN-U- 211 158 664
- CN-U- 213 475 846
- CN-U- 216 614 471
- CN-U- 218 910 200
- US-A1- 2009 007 903

## Description

### TECHNICAL FIELD

The present disclosure relates to the field of xylitol preparation, and in particular to a preparation system and a preparation method for xylitol crystals.

### BACKGROUND

The existing xylitol production process uses corn cobs as raw materials to obtain xylitol crystals through hydrolysis, hydrogenation, decolorization, ion exchange, concentration, crystallization, centrifugation, drying, etc. The crystallization determines the quality and yield of xylitol products, which plays a decisive role in the morphology, purity, bulk density, particle size distribution, and caking properties of the crystal products.

The thrust of the xylitol crystallization process mainly comes from the thermodynamically non-equilibrium properties of the crystallization system. Solubility, mesa-stable region, induction period, and other crystallization thermodynamic properties have a great impact on the selection of the crystallization manner and the crystallization yield. At present, the crystallization manner commonly used in the industry includes evaporation crystallization and cooling crystallization. Both evaporation crystallization and cooling crystallization are well applied to the crystallization process of xylitol due to the large solubility of the xylitol.

However, as for evaporation crystallization or cooling crystallization, due to unreasonable process parameters, or the lack of theoretical and factual basis for the time of adding crystal seeds when adding the crystal seeds based on experience, problems such as a time-consuming xylitol crystallization process, severe secondary nucleation, high fine powder content, significant product variations between different batches, and unstable crystallization process exist, which leads to caking of xylitol products. For example, the patent publication No. CN1736970A discloses a method for refining and crystallizing xylitol. By optimizing a speed, a count of seeds, a particle size of seeds, a cooling rate and other factors of the crystallization process, although xylitol crystals with concentrated and adjustable particle size are obtained, it is impossible to add 3~6% seeds in production, which limits the application of the process in production. As another example, the patent publication No. CN102731252A discloses a crystallization method of xylitol or maltitol. By adding a small amount of anti-caking agent during the crystallization process, although the crystal caking phenomenon is effectively alleviated, the anti-caking agent may have an impact on product quality, and the method of adding anti-caking agents is not allowed in relevant standards. CN 114478191 discloses a process and a system for the purification of xylitol comprising decolorisation, ion exchange evaporation, crystallisation and centrifugation followed by further treatment of the mother liquor through an analogous sequence of step including nanofiltration.

Therefore, from the perspective of industrial implementation, optimizing the crystallization process parameters to reduce the phenomenon of secondary nucleation and control the particle size distribution of the product has become one of the most important measures to avoid or alleviate the caking of the xylitol crystals.

### SUMMARY

A technical problem to be solved by the present disclosure is to provide a preparation system and preparation method for xylitol crystals, which can increase the particle size of xylitol crystal products and prolong the caking cycle of xylitol crystal products.

The present disclosure is implemented by providing a preparation system for xylitol crystals. The preparation system comprises a blending tank, a decolorization tank, an ion exchange column, a nanofiltration system, a first evaporator, a first crystallization kettle, a first centrifuge, a hot air drying tank, and a first fluidized bed dryer that are sequentially connected through pipelines; a primary mother liquor storage tank, a second evaporator, a second crystallization kettle, a second centrifuge, a second fluidized bed dryer, and a dicrystalline sugar dissolution tank that are sequentially connected through pipelines; and a secondary mother liquor storage tank, a third evaporator, a third crystallization kettle, a third centrifuge, a third fluidized bed dryer, and a tricrystalline sugar dissolution tank that are sequentially connected through pipelines. A solid outlet of the first centrifuge is connected with an inlet of the hot air drying tank through a pipeline. A liquid outlet of the first centrifuge is connected with an inlet of the primary mother liquor storage tank through a pipeline. A solid outlet of the second centrifuge is connected with an inlet of the second fluidized bed dryer through a pipeline. A liquid outlet of the second centrifuge is connected with an inlet of the secondary mother liquor storage tank through a pipeline. The dicrystalline sugar dissolution tank is provided with a water inlet for purified water. An outlet of the dicrystalline sugar dissolution tank is connected with an inlet of the blending tank. The tricrystalline sugar dissolution tank is also provided with a water inlet for purified water. An outlet of the tricrystalline sugar dissolution tank is connected with an inlet of the primary mother liquor storage tank through a pipeline. The blending tank is provided with a feed pipe for a hydrogenation solution. A raw material of xylitol hydrogenation solution for preparing the xylitol crystals is stored in the blending tank. The blending tank is used for mixing the raw material of xylitol hydrogenation solution with a dicrystalline sugar solution delivered from the dicrystalline sugar dissolution tank. An output material of an outlet of the first fluidized bed dryer is prepared xylitol crystals.

The preparation system may further include a liquid xylitol storage tank, wherein an outlet of the third centrifuge is connected with an inlet of the liquid xylitol storage tank through a pipeline.

The present disclosure is implemented by providing a preparation method for xylitol crystals. The preparation method is performed using the preparation system for the xylitol crystals. The preparation method includes:
operation 1, in a blending tank, blending the raw material of xylitol hydrogenation solution with the dicrystalline sugar solution in a certain ratio, and then sequentially passing the raw material of xylitol hydrogenation solution blended with the dicrystalline sugar solution through the decolorization tank for decolorization treatment, the ion exchange column for impurity removal treatment, the nanofiltration system for filtration treatment, and the first evaporator for evaporation and concentration treatment to obtain xylitol concentrate, wherein a refractive index of the xylitol concentrate is within a range of 78-82%, a temperature of the xylitol concentrate is within a range of 90-100°C, and a conductivity of the xylitol concentrate is smaller than 20 µs/cm;
operation 2, entering the xylitol concentrate into the first crystallization kettle through a pipeline, including: feeding the first crystallization kettle with an initial volume of xylitol concentrate, controlling a vacuum degree of the first crystallization kettle to be -0.095 MPa to and a temperature of the first crystallization kettle to be 65 °C, and adding an appropriate amount of xylitol crystal seeds to stimulate crystallization until xylitol crystals begin to crystalize in the first crystallization kettle, adding an additional volume of xylitol concentrate to the first crystallization kettle, stirring for 7-12 h, and at an end of crystallization, feeding steam to increase a system temperature to 66°C -68 °C and maintaining the system temperature for a certain period of time to obtain a xylitol sugar paste, wherein a ratio of the initial volume to the additional volume is within a range of 1:0.8-1.5, the stirring is a variable frequency stirring, a timing of adding the xylitol crystal seeds is that a count of particles of the xylitol crystals observed from a view window of the first crystallization kettle is less than 50, and the certain period of time for maintaining the system temperature at the end of crystallization is 0.5~2.0h;
operation 3, performing, using the first centrifuge, a separation treatment on the xylitol sugar paste obtained in operation 2 to obtain crystalline xylitol and a primary mother liquor, entering the primary mother liquor into the primary mother liquor tank through a pipeline for temporary storage, and passing the crystalline xylitol through the hot air drying tank for drying treatment and the first fluidized bed dryer for cold air drying treatment to obtain the prepared xylitol crystals; sequentially passing the primary mother liquor through the second evaporator for concentration treatment, the second crystallization kettle for crystallization treatment, and the second centrifuge for centrifugation treatment to obtain dicrystalline sugar and a secondary mother liquor, and entering the secondary mother liquor into the secondary mother liquor tank through a pipeline for temporary storage; passing the dicrystalline sugar through the second fluidized bed dryer for drying treatment and the dicrystalline sugar dissolution tank for dissolution treatment, inputting the purified water to dissolve the dicrystalline sugar to obtain a dicrystalline sugar solution, and entering the dicrystalline sugar solution into the blending tank through a pipeline for blending with the raw material of xylitol hydrogenation solution; and
operation 4, sequentially passing the secondary mother liquor obtained in operation 3 through the third evaporator for concentration treatment, the third crystallization kettle for crystallization treatment, and the third centrifuge for centrifugation treatment to obtain tricrystalline sugar and a tertiary mother liquor, passing the tricrystalline sugar through the third fluidized bed dryer for drying treatment and the tricrystalline sugar dissolution tank for dissolution treatment, inputting the purified water to dissolve the tricrystalline sugar to obtain a tricrystalline sugar solution, and entering the tricrystalline sugar solution into the primary mother liquor tank through a pipeline to blend the tricrystalline sugar solution with the primary mother liquor for reuse, the tertiary mother liquor being stored as liquid xylitol.

Compared with the existing techniques, the preparation system and preparation method for xylitol crystals of the present disclosure have the following advantages: xylitol crystals with larger particle size and more concentrated particle size distribution can be obtained, wherein in the prepared xylitol crystals, a proportion of 30-mesh xylitol particles can be increased by more than 30%, and the results of small package samples show that the caking cycle of the xylitol crystal products has increased from 45 days to more than 100 days.

### BRIEF DESCRIPTION OF THE DRAWINGS

FIG. 1 is a schematic diagram illustrating a principle of a preparation system for xylitol crystals according to some embodiments of the present disclosure; and

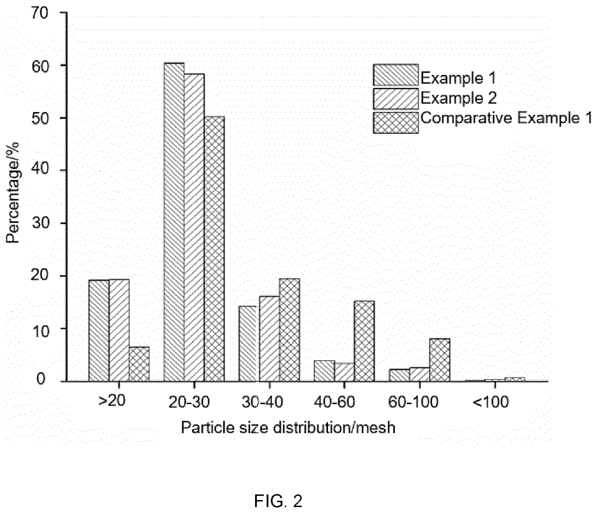

FIG. 2 is a schematic diagram illustrating a particle size distribution of xylitol crystals prepared in some embodiments of the present disclosure and Comparative Examples.

### DETAILED DESCRIPTION

In order to more clearly illustrate the technical problems to be solved by the present disclosure, technical solutions, and beneficial effects, the present disclosure will be further illustrated in the description of the embodiments with reference to the accompanying drawings. It should be understood that the specific embodiments described herein are only intended to explain the present disclosure and are not intended to limit the present disclosure.

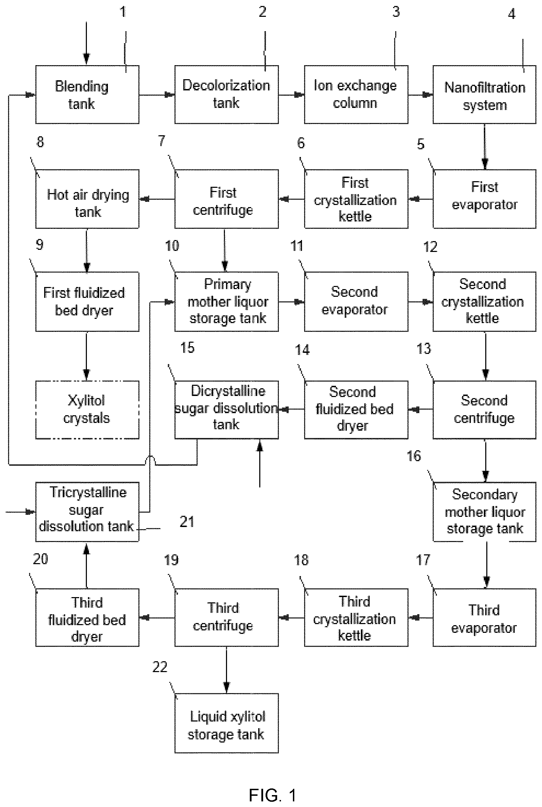

As illustrated in FIG. 1, the preparation system for the xylitol crystals in a preferred embodiment comprises a blending tank 1, a decolorization tank 2, an ion exchange column 3, a nanofiltration system 4, a first evaporator 5, a first crystallization kettle 6, a first centrifuge 7, a hot air drying tank 8, and a first fluidized bed dryer 9 that are sequentially connected through pipelines; a primary mother liquor storage tank 10, a second evaporator 11, a second crystallization kettle 12, a second centrifuge 13, a second fluidized bed dryer 14, and a dicrystalline sugar dissolution tank 15 that are sequentially connected through pipelines; and a secondary mother liquor storage tank 16, a third evaporator 17, a third crystallization kettle 18, a third centrifuge 19, a third fluidized bed dryer 20, and a tricrystalline sugar dissolution tank 21 that are sequentially connected through pipelines. Arrows in the figure indicate a material flow direction in the system.

A solid outlet of the first centrifuge 7 is connected with an inlet of the hot air drying tank 8 through a pipeline. A liquid outlet of the first centrifuge 7 is connected with an inlet of the primary mother liquor storage tank 10 through a pipeline. A solid outlet of the second centrifuge 13 is connected with an inlet of the second fluidized bed dryer 14 through a pipeline. A liquid outlet of the second centrifuge 13 is connected with an inlet of the secondary mother liquor storage tank 16 through a pipeline. The dicrystalline sugar dissolution tank 15 is provided with a water inlet for purified water. An outlet of the dicrystalline sugar dissolution tank 15 is connected with an inlet of the blending tank 1 through a pipeline. The tricrystalline sugar dissolution tank 21 is also provided with a water inlet for purified water. An outlet of the tricrystalline sugar dissolution tank 21 is connected with the inlet of the primary mother liquor storage tank 10 through a pipeline. The blending tank 1 is also provided with a feed pipe for a hydrogenation solution. A raw material of xylitol hydrogenation solution for preparing the xylitol crystals is stored in the blending tank. The blending tank 1 is configured to mix a raw material of xylitol hydrogenation solution with a dicrystalline sugar solution delivered from the dicrystalline sugar dissolution tank 15. An output material of an outlet of the first fluidized bed dryer 9 is prepared xylitol crystals.

The preparation system further comprises a liquid xylitol storage tank 22. An outlet of the third centrifuge 19 is connected with an inlet of the liquid xylitol storage tank 22 through a pipeline.

Referring to FIG. 1, the embodiments of the present disclosure further disclose a preparation method for xylitol crystals. One or more operations in a process of the method are performed using the preparation system for the xylitol crystals as illustrated in the aforementioned embodiments. Process may include the following operations.

Operation 1, in the blending tank 1, a raw material of xylitol hydrogenation solution is blended with a dicrystalline sugar solution in a certain ratio, and then the raw material of xylitol hydrogenation solution blended with the dicrystalline sugar solution is passed through the decolorization tank 2 for decolorization treatment, the ion exchange column 3 for impurity removal treatment, the nanofiltration system for filtration treatment, and the first evaporator 5 for evaporation and concentration treatment to obtain xylitol concentrate. A refractive index of the xylitol concentrate is within a range of 78-82%, a temperature of the xylitol concentrate is within a range of 90-100°C, and a conductivity of the xylitol concentrate is smaller than20 µs/cm.

Operation 2, the xylitol concentrate enters the first crystallization kettle 6 through a pipeline. Specifically, the first crystallization kettle 6 is first fed with an initial volume of xylitol concentrate. A vacuum degree of the first crystallization kettle 6 is -0.095 MPa and a temperature of the first crystallization kettle 6 is 65 °C, and an appropriate amount of xylitol crystal seeds is added to stimulate crystallization until xylitol crystals begin to crystallize in the first crystallization kettle 6. At the same time, an additional volume of xylitol concentrate is added to the first crystallization kettle 6, and stirring is performed for 7-12 h. At the end of crystallization, steam is fed to increase a system temperature to be 66°C -68 °C and the system temperature is maintained for a certain period of time to obtain a xylitol sugar paste. The stirring is a variable frequency stirring, a timing of adding the xylitol crystal seeds is that a count of particles of the xylitol crystals observed from a view window of the first crystallization kettle is less than 50, and the certain period of time for maintaining the system temperature at the end of crystallization is 0.5-2.0 h.

Operation 3, a separation treatment is performed, using the first centrifuge 7, on the xylitol sugar paste obtained in operation 2 to obtain crystalline xylitol and a primary mother liquor. The primary mother liquor may enter the primary mother liquor tank 10 through a pipeline for temporary storage. The crystalline xylitol is passed through the hot air drying tank 8 for drying treatment and the first fluidized bed dryer 9 for cold air drying treatment to obtain a xylitol crystal product (i.e., prepared xylitol crystals). The primary mother liquor sequentially passes the second evaporator 11 for concentration treatment, the second crystallization kettle 12 for crystallization treatment, and the second centrifuge 13 for centrifugation treatment to obtain dicrystalline sugar and a secondary mother liquor. The secondary mother liquor enters the secondary mother liquor tank 16 through a pipeline for temporary storage. The dicrystalline sugar passes through the second fluidized bed dryer 14 for drying treatment and then the dicrystalline sugar dissolution tank 15 for a dissolution treatment. Purified water is input into the dicrystalline sugar dissolution tank to dissolve the dicrystalline sugar to obtain a dicrystalline sugar solution. The dicrystalline sugar solution enters the blending tank 1 through a pipeline to be blended with the raw material of xylitol hydrogenation solution.

Operation 4, the secondary mother liquor obtained in operation 3 sequentially passes through the third evaporator 17 for concentration treatment, the third crystallization kettle 18 for crystallization treatment, and the third centrifuge 19 for centrifugation treatment to obtain tricrystalline sugar and a tertiary mother liquor. The tricrystalline sugar passes through the third fluidized bed dryer 20 for drying treatment and then the tricrystalline sugar dissolution tank 21 for dissolution treatment. The purified water is input into the tricrystalline sugar dissolution tank to dissolve the tricrystalline sugar to obtain a tricrystalline sugar solution. The tricrystalline sugar solution enters the primary mother liquor tank 10 through a pipeline to be blended with the primary mother liquor for reuse. The tertiary mother liquor is stored as liquid xylitol.

In operation 1, a size of a nanofiltration membrane of the nanofiltration system 4 is within a range of 300-800 Da.

In operation 2, the first crystallization kettle 6 is a vacuum evaporation crystallization kettle. A stirring speed in the variable frequency stirring gradually reduces from 90 rpm to 10 rpm, a size of the view window of the first crystallization kettle is 1 cm², a count of particles of the xylitol crystals is observed from the view window using a 10 x 10 magnification microscope, and the microscope is configured to observe a count of particles of the xylitol crystals through the view window an amount of the xylitol crystal seeds accounts 0.0004-0.0008% of a mass of a solution in the first crystallization kettle 6, and a particle size of the xylitol crystal seeds is within a range of 80-100 mesh.

In operation 2, the xylitol sugar paste is sprayed and washed for 5-10s using an 80-100% concentration ethanol solution after the separation treatment is performed on the xylitol sugar paste using the first centrifuge 7 to remove as much water as possible from the crystal xylitol.

In operation 3, a temperature of the hot air of the hot air drying tank 8 is within a range of 75-85°C.

The preparation system for the xylitol crystal is further illustrated through specific Examples below.

### Example 1

A preparation method for xylitol crystals of some embodiments of the present disclosure comprises the following operations.

Operation 11, a xylitol hydrogenation solution was blended with a dicrystalline sugar solution in a certain ratio, and then the xylitol hydrogenation solution blended with the dicrystalline sugar solution sequentially passed through the decolorization tank 2 for activated carbon decolorization treatment, the ion exchange column 3 for ion exchange treatment, a 400 Da nanofiltration system for treatment, and the first evaporator 5 for treatment to obtain xylitol concentrate with a refractive index of 79%, a temperature of 95 °C, and a conductivity of 1.958 µs/cm.

Operation 12, 10 tons of xylitol concentrate was fed into the first crystallization kettle 6 to be further concentrated at -0.095 MPa until a system temperature was cooled down to about 65 °C and 20 fine crystals were observed in a view window of the first crystallization kettle 6, then 0.0005% of xylitol crystal seeds were added to stimulate crystallization, at the same time 10 tons of xylitol concentrate was added to the first crystallization kettle 6. With a constant vacuum degree of -0.095 MPa and a constant temperature of 65 °C, the solution in the first crystallization kettle 6 was stirred at an initial speed of 90 rpm with variable frequency and crystallized. After 7 h of crystallization, steam was fed to increase the system temperature to 67 °C and then the system temperature 67 °C was maintained for 0.5 h for crystallization to obtain a xylitol sugar paste.

Operation 13, a separation treatment was performed on the obtained xylitol sugar paste by the first centrifuge 7 to obtain 8.7 tons of crystalline xylitol and 10.5 tons of primary mother liquor. The crystalline xylitol was dried by 80 °C hot air of the hot air drying tank 8 and dried by the cold air of the first fluidized bed dryer 9 to obtain a xylitol crystal product. The primary mother liquor sequentially passed through the second evaporator 11 for concentration treatment, the second crystallization kettle 12 for crystallization treatment at the vacuum degree of -0.095 MPa and the temperature of 65 °C, and the second centrifuge 13 for centrifugation treatment to obtain dicrystalline sugar and a secondary mother liquor. The dicrystalline sugar was dried by the second fluidized bed dryer 14 and then entered the dicrystalline sugar dissolution tank 15 to be dissolved to obtain a dicrystalline sugar solution. Then the dicrystalline sugar solution was delivered to the blending tank 1 to be blended and mixed with the xylitol hydrogenation solution. The secondary mother liquor sequentially passed through the third evaporator 17 for concentration treatment, the third crystallization kettle 18 for crystallization treatment, and the third centrifuge 19 for centrifugation treatment to obtain tricrystalline sugar and a tertiary mother liquor. The tertiary mother liquor was stored in the liquid xylitol storage tank 22. The tricrystalline sugar was dried by the third fluidized bed dryer 20 and then entered the tricrystalline sugar dissolution tank 21 to be dissolved to obtain a tricrystalline sugar solution. The tricrystalline sugar solution entered the primary mother liquor tank 10 through a pipeline to be blended with the primary mother liquor for reuse.

The prepared xylitol crystal product was sieved to obtain a particle size distribution of the xylitol crystal product shown in Table 1.

**Table 1 The particle size distribution of the xylitol crystal product prepared in Example 1**

| Particle size/mesh | >20 | 20-30 | 30-40 | 40-60 | 60-100 | <100 |
|---|---|---|---|---|---|---|
| Percentage/% | 19.17 | 60.38 | 14.19 | 3.95 | 2.26 | 0.05 |

A caking cycle of a small packaged sample of xylitol crystals prepared in Example 1 is 105 days

### Example 2

Another preparation method for xylitol crystals of some embodiments of the present disclosure comprises the following operations.

Operation 21, a xylitol hydrogenation solution was blended with a dicrystalline sugar solution in a certain ratio, and then the xylitol hydrogenation solution blended with the dicrystalline sugar solution sequentially passed through the decolorization tank 2 for decolorization treatment, the ion exchange column 3 for ion exchange treatment, the 400 Da nanofiltration system 4 for treatment, and the first evaporator 5 for treatment to obtain xylitol concentrate with a refractive index of 80%, a temperature of 95°C, and a conductivity of 1.744 µs/cm.

Operation 22, 10 tons of xylitol concentrate was fed into the first crystallization kettle 6 to be further concentrated at -0.095 MPa until a system temperature was cooled down to about 65 °C and 25 fine crystals were observed in a view window of the first crystallization kettle 6, then 0.0005% of xylitol crystal seeds were added to stimulate crystallization, at the same time 10 tons of xylitol concentrate was added to the first crystallization kettle 6. With a constant vacuum degree of -0.095 MPa and the constant temperature of 65 °C of the system, the solution in the first crystallization kettle 6 was stirred at an initial speed of 90 rpm with variable frequency and crystallized. After 8 h of crystallization, steam was fed to increase the system temperature to 66.5 °C and then the system temperature 66.5 °C was maintained for 0.8 h for crystallization to obtain a xylitol sugar paste.

Operation 23, a separation treatment was performed on the obtained xylitol sugar paste by the first centrifuge 7 to obtain 8.6 tons of crystalline xylitol and 10.6 tons of primary mother liquor. The crystalline xylitol was dried by 80 °C hot air of the hot air drying tank 8 and dried by the cold air of the first fluidized bed dryer 9 to obtain a xylitol crystal product. The primary mother liquor sequentially passed through the second evaporator 11 for concentration treatment, the second crystallization kettle 12 for crystallization treatment at the vacuum degree of -0.095 MPa and the temperature of 65 °C, and the second centrifuge 13 for centrifugation treatment to obtain dicrystalline sugar and a secondary mother liquor. The dicrystalline sugar was dried by the second fluidized bed dryer 14 and then entered the dicrystalline sugar dissolution tank 15 to be dissolved to obtain a dicrystalline sugar solution. Then the dicrystalline sugar solution was delivered to the blending tank 1 to be blended and mixed with the xylitol hydrogenation solution. The secondary mother liquor sequentially passed through the third evaporator 17 for concentration treatment, the third crystallization kettle 18 for crystallization treatment, and the third centrifuge 19 for centrifugation treatment to obtain tricrystalline sugar and a tertiary mother liquor. The tertiary mother liquor was stored in the liquid xylitol storage tank 22. The tricrystalline sugar was dried by the third fluidized bed dryer 20 and then entered the tricrystalline sugar dissolution tank 21 to be dissolved to obtain a tricrystalline sugar solution. The tricrystalline sugar solution entered the primary mother liquor tank 10 through a pipeline to be blended with the primary mother liquor for reuse.

The prepared xylitol crystal product was sieved to obtain a particle size distribution of the xylitol crystal product shown in Table 2.

**Table 2 The particle size distribution of the xylitol crystal product prepared in Example 2**

| Particle size/mesh | >20 | 20-30 | 30-40 | 40-60 | 60-100 | <100 |
|---|---|---|---|---|---|---|
| Percentage/% | 19.28 | 58.31 | 16.11 | 3.40 | 2.57 | 0.33 |

A caking cycle of a small packaged sample of xylitol crystals prepared in Example 2 is 112 days.

To better describe the beneficial effects brought about by the present disclosure, the present disclosure is further illustrated with reference to the following Comparative Example.

### Comparative Example

The Comparative Example of the present disclosure comprises the following operations.

Operation 31, a xylitol hydrogenation solution was blended with a primary centrifugal mother liquor in a certain ratio, and then a decolorization treatment by activated carbon, ion exchange, and triple effect evaporation were performed, respectively, to obtain xylitol concentrate with a refractive index of 80.5%, a temperature of 95°C, and a conductivity of 2.063 µs/cm.

Operation 32, 7 tons of xylitol concentrate was fed into a crystallization tank to be further concentrated at -0.095 MPa until a system temperature was cooled down to about 65 °C and a large number (>50) of fine crystals were observed in a view window of the crystallization tank, then 0.0005% of xylitol crystal seeds were added to stimulate crystallization, at the same time 13 tons of xylitol concentrate was added to the crystallization tank. With a constant vacuum degree of -0.095 MPa and a constant temperature of 65 °C of the system, the solution in the crystallization tank was stirred at a constant speed of 90 rpm and crystallized for 8 h to obtain a xylitol sugar paste.

Operation 33, the obtained xylitol sugar paste was separated by a high-speed centrifuge to obtain 8.8 tons of xylitol crystals and 10.2 tons of primary centrifugal mother liquor. The xylitol crystals were dried by 88 °C hot air and dried by cold air to obtain a xylitol product. The primary centrifugal mother liquor was directly returned to be blended with the xylitol hydrogenation solution.

The prepared xylitol crystal product was sieved to obtain a particle size distribution of the xylitol crystal product shown in Table 3.

**Table 3 The particle size distribution of the xylitol crystal product prepared in Comparative Example**

| Particle size/mesh | 20 | 20-30 | 30-40 | 40-60 | 60-100 | 100 |
|---|---|---|---|---|---|---|
| Percentage/% | 6.47 | 50.21 | 19.42 | 15.19 | 8.04 | 0.67 |

A caking cycle of a small packaged sample of xylitol crystals prepared in Comparative Example is 45 days.

Compared with the Comparative Example, Example 1 and Example 2 obtained xylitol crystal products with larger particles and narrower particle size distributions by reducing returning of the xylitol mother liquor, adding the nanofiltration system to remove low-molecular-weight impurities, optimizing the crystallization process, adopting an alcoholic washing in the centrifugation process, etc. In addition, the caking cycle of the xylitol crystal products was significantly improved. The comparison data is shown in Table 4 and FIG. 4.

**Table 4 Comparison table of assay data for the xylitol crystal product prepared in each of Examples with the xylitol crystal product prepared in Comparative Example**

| Item | Percentage of particle size >30 mesh/% | Sample days of small packaged sample/day |
|---|---|---|
| Example 1 | 79.55 | 105 |
| Example 2 | 77.59 | 112 |
| Comparative Example | 56.68 | 45 |

It can be seen from the above table that in the comparison item of the percentage of particle size >30 mesh, the xylitol crystalline product prepared in Example 1 is 40% higher than that of Comparative Example, and the xylitol crystalline product prepared in Example 2 is 36% higher than that of the Comparative Example. In addition, the caking cycles of the xylitol crystal products prepared in Example 1 and Example 2 are significantly higher than that of Comparative Example.

The above descriptions are only preferred embodiments of the present disclosure and are not intended to limit the present disclosure.

## Claims

1. A preparation system for xylitol crystals, comprising:
a blending tank, a decolorization tank, an ion exchange column, a nanofiltration system, a first evaporator, a first crystallization kettle, a first centrifuge, a hot air drying tank, and a first fluidized bed dryer that are sequentially connected through pipelines;
a primary mother liquor storage tank, a second evaporator, a second crystallization kettle, a second centrifuge, a second fluidized bed dryer, and a dicrystalline sugar dissolution tank that are sequentially connected through pipelines; and
a secondary mother liquor storage tank, a third evaporator, a third crystallization kettle, a third centrifuge, a third fluidized bed dryer, and a tricrystalline sugar dissolution tank that are sequentially connected through pipelines, wherein
a solid outlet of the first centrifuge is connected with an inlet of the hot air drying tank through a pipeline, a liquid outlet of the first centrifuge is connected with an inlet of the primary mother liquor storage tank through a pipeline, a solid outlet of the second centrifuge is connected with an inlet of the second fluidized bed dryer through a pipeline, a liquid outlet of the second centrifuge is connected with an inlet of the secondary mother liquor storage tank through a pipeline, the dicrystalline sugar dissolution tank is provided with a water inlet for purified water, an outlet of the dicrystalline sugar dissolution tank is connected with an inlet of the blending tank, the tricrystalline sugar dissolution tank is provided with a water inlet for purified water, an outlet of the tricrystalline sugar dissolution tank is connected with the inlet of the primary mother liquor storage tank through a pipeline, the blending tank is provided with a feed pipe for a hydrogenation solution, a raw material of xylitol hydrogenation solution for preparing the xylitol crystals is stored in the blending tank, the blending tank is configured to mix the raw material of xylitol hydrogenation solution with a dicrystalline sugar solution delivered from the dicrystalline sugar dissolution tank, and an output of an outlet of the first fluidized bed dryer is prepared xylitol crystals.

2. The preparation system of claim 1, further comprising a liquid xylitol storage tank, wherein an outlet of the third centrifuge is connected with an inlet of the liquid xylitol storage tank through a pipeline.

3. A preparation method for xylitol crystals, performed using the preparation system for the xylitol crystals of claim 1 or claim 2, wherein the preparation method comprises:
operation 1, in the blending tank, blending the raw material of xylitol hydrogenation solution with the dicrystalline sugar solution in a certain ratio, and then sequentially passing the raw material of xylitol hydrogenation solution blended with the dicrystalline sugar solution through the decolorization tank for decolorization treatment, the ion exchange column for impurity removal treatment, the nanofiltration system for filtration treatment, and the first evaporator for evaporation and concentration treatment to obtain xylitol concentrate, wherein a refractive index of the xylitol concentrate is within a range of 78-82%, a temperature of the xylitol concentrate is within a range of 90-100°C, and a conductivity of the xylitol concentrate is smaller than 20 µs/cm;
operation 2, entering the xylitol concentrate into the first crystallization kettle through a pipeline, including: feeding the first crystallization kettle with an initial volume of xylitol concentrate, controlling a vacuum degree of the first crystallization kettle to be -0.095 MPa and a temperature of the first crystallization kettle to be 65 °C, and adding an appropriate amount of xylitol crystal seeds to stimulate crystallization until xylitol crystals begin to crystalize in the first crystallization kettle, adding an additional volume of xylitol concentrate to the first crystallization kettle, stirring for 7-12 h, and at an end of crystallization, feeding steam to increase a system temperature to 66°C -68 °C and maintaining the system temperature for a certain period of time to obtain a xylitol sugar paste, wherein a ratio of the initial volume to the additional volume is within a range of 1:0.8-1.5, the stirring is a variable frequency stirring, a timing of adding the xylitol crystal seeds is that a count of particles of the xylitol crystals observed from a view window of the first crystallization kettle is less than 50, and the certain period of time for maintaining the system temperature at the end of crystallization is 0.5~2.0 h;
operation 3, performing, using the first centrifuge, a separation treatment on the xylitol sugar paste obtained in operation 2 to obtain crystalline xylitol and a primary mother liquor, entering the primary mother liquor into the primary mother liquor tank through a pipeline for temporary storage, and passing the crystalline xylitol through the hot air drying tank for drying treatment and the first fluidized bed dryer for cold air drying treatment to obtain the prepared xylitol crystals; sequentially passing the primary mother liquor through the second evaporator for concentration treatment, the second crystallization kettle for crystallization treatment, and the second centrifuge for centrifugation treatment to obtain dicrystalline sugar and a secondary mother liquor, and entering the secondary mother liquor into the secondary mother liquor tank through a pipeline for temporary storage; passing the dicrystalline sugar through the second fluidized bed dryer for drying treatment and the dicrystalline sugar dissolution tank for dissolution treatment, inputting the purified water to dissolve the dicrystalline sugar to obtain a dicrystalline sugar solution, and entering the dicrystalline sugar solution into the blending tank through a pipeline for blending with the raw material of xylitol hydrogenation solution; and
operation 4, sequentially passing the secondary mother liquor obtained in operation 3 through the third evaporator for concentration treatment, the third crystallization kettle for crystallization treatment, and the third centrifuge for centrifugation treatment to obtain tricrystalline sugar and a tertiary mother liquor, passing the tricrystalline sugar through the third fluidized bed dryer for drying treatment and the tricrystalline sugar dissolution tank for dissolution treatment, inputting the purified water to dissolve the tricrystalline sugar to obtain a tricrystalline sugar solution, and entering the tricrystalline sugar solution into the primary mother liquor tank through a pipeline to blend the tricrystalline sugar solution with the primary mother liquor for reuse, the tertiary mother liquor being stored as liquid xylitol.

4. The preparation method of claim 3, wherein in operation 1, a size of a nanofiltration membrane of the nanofiltration system is within a range of 300-800 Da.

5. The preparation system of claim 1 and preparation method of claim 3, wherein in operation 2, the first crystallization kettle is a vacuum evaporation crystallization kettle, a stirring speed in the variable frequency stirring gradually reduces from 90 rpm to 10 rpm, a size of the view window of the first crystallization kettle is 1 cm², the count of particles of the xylitol crystals is observed from the view window using a 10 x 10 magnification microscope, and the microscope is configured to observe a count of particles of the xylitol crystals through the view window, an amount of the xylitol crystal seeds accounts 0.0004-0.0008% of a mass of a solution in the first crystallization kettle, and a particle size of the xylitol crystal seeds is within a range of 80-100 mesh.

6. The preparation method of claim 3, wherein in operation 2, the xylitol sugar paste is sprayed and washed for 5-10 s using an 80-100% concentration ethanol solution after the separation treatment is performed on the xylitol sugar paste using the first centrifuge, so as to remove as much water as possible from the crystalline xylitol.

7. The preparation method of claim 3, wherein in operation 3, a temperature of a hot air of the hot air drying tank is within a range of 75-85°C.

## Patentansprüche

1. Herstellungssystem für Xylitkristalle, umfassend:
einen Mischbehälter, einen Entfärbungsbehälter, eine Ionenaustauschkolonne, ein Nanofiltrationssystem, einen ersten Verdampfer, einen ersten Kristallisationskessel, eine erste Zentrifuge, einen Heißlufttrocknungsbehälter und einen ersten Wirbelschichttrockner, die der Reihe nach über Rohrleitungen miteinander verbunden sind;
einen Speicherbehälter für Primärmutterlauge, einen zweiten Verdampfer, einen zweiten Kristallisationskessel, eine zweite Zentrifuge, einen zweiten Wirbelschichttrockner und einen Auflösebehälter für zweikristallinen Zucker, die der Reihe nach über Rohrleitungen miteinander verbunden sind; und
einen Speicherbehälter für Sekundärmutterlauge, einen dritten Verdampfer, einen dritten Kristallisationskessel, eine dritte Zentrifuge, einen dritten Wirbelschichttrockner und einen Auflösebehälter für dreikristallinen Zucker, die der Reihe nach über Rohrleitungen miteinander verbunden sind, wobei
ein Feststoffauslass der ersten Zentrifuge über eine Rohrleitung mit einem Einlass des Heißlufttrocknungsbehälters verbunden ist, ein Flüssigkeitsauslass der ersten Zentrifuge über eine Rohrleitung mit einem Einlass des Speicherbehälters für Primärmutterlauge verbunden ist, ein Feststoffauslass der zweiten Zentrifuge über eine Rohrleitung mit einem Einlass des zweiten Wirbelschichttrockners verbunden ist, ein Flüssigkeitsauslass der zweiten Zentrifuge über eine Rohrleitung mit einem Einlass des Speicherbehälters für Sekundärmutterlauge verbunden ist, der Auflösebehälter für zweikristallinen Zucker mit einem Wassereinlass für gereinigtes Wasser versehen ist, ein Auslass des Auflösebehälters für zweikristallinen Zucker mit einem Einlass des Mischbehälters verbunden ist, der Auflösebehälter für dreikristallinen Zucker mit einem Wassereinlass für gereinigtes Wasser versehen ist, ein Auslass des Auflösebehälters für dreikristallinen Zucker über eine Rohrleitung mit dem Einlass des Speicherbehälters für Primärmutterlauge verbunden ist, der Mischbehälter mit einer Zufuhrleitung für eine Hydrierlösung versehen ist, ein Rohmaterial einer Xylit-Hydrierungslösung zur Herstellung der Xylitkristalle in dem Mischbehälter gespeichert ist, der Mischbehälter dazu ausgebildet ist, das Rohmaterial der Xylit-Hydrierungslösung mit einer aus dem Auflösebehälter für zweikristallinen Zucker zugeführten Zweikristallzuckerlösung zu mischen, und ein Auslass des ersten Wirbelschichttrockners hergestellte Xylitkristalle ausgibt.

2. Herstellungssystem nach Anspruch 1, ferner umfassend einen Speicherbehälter für flüssiges Xylit, wobei ein Auslass der dritten Zentrifuge über eine Rohrleitung mit einem Einlass des Speicherbehälters für flüssiges Xylit verbunden ist.

3. Herstellungsverfahren für Xylitkristalle, ausgeführt unter Verwendung des Herstellungssystems für die Xylitkristalle nach Anspruch 1 oder Anspruch 2, wobei das Herstellungsverfahren umfasst:
Vorgang 1, in dem Mischbehälter das Rohmaterial der Xylit-Hydrierungslösung und die Zweikristallzuckerlösung in einem bestimmten Verhältnis zu vermischen und anschließend das mit der Zweikristallzuckerlösung vermischte Rohmaterial der Xylit-Hydrierungslösung der Reihe nach durch den Entfärbungsbehälter zur Entfärbungsbehandlung, die Ionenaustauschkolonne zur Verunreinigungsentfernung, das Nanofiltrationssystem zur Filtrationsbehandlung und den ersten Verdampfer zur Verdampfungs- und Konzentrationsbehandlung zu leiten, um ein Xylitkonzentrat zu erhalten, wobei ein Brechungsindex des Xylitkonzentrats im Bereich von 78-82% liegt, eine Temperatur des Xylitkonzentrats im Bereich von 90-100°C liegt und eine Leitfähigkeit des Xylitkonzentrats kleiner als 20 µs/cm ist;
Vorgang 2, das Xylitkonzentrat über eine Rohrleitung in den ersten Kristallisationskessel einzuleiten, umfassend: den ersten Kristallisationskessel mit einem Anfangsvolumen an Xylitkonzentrat zu beschicken, einen Vakuumgrad des ersten Kristallisationskessels auf -0.095 MPa und eine Temperatur des ersten Kristallisationskessels auf 65°C zu regeln und eine geeignete Menge Xylit-Kristallsaat zuzusetzen, um die Kristallisation anzuregen, bis Xylitkristalle im ersten Kristallisationskessel zu kristallisieren beginnen, ein zusätzliches Volumen an Xylitkonzentrat in den ersten Kristallisationskessel zuzugeben, 7-12 h zu rühren und am Ende der Kristallisation Dampf zuzuführen, um eine Systemtemperatur auf 66°C-68°C zu erhöhen und die Systemtemperatur für einen bestimmten Zeitraum aufrechtzuerhalten, um eine Xylit-Zuckerpaste zu erhalten, wobei ein Verhältnis des Anfangsvolumens zu dem zusätzlichen Volumen im Bereich von 1:0.8-1.5 liegt, das Rühren ein frequenzvariables Rühren ist, ein Zeitpunkt des Zugebens der Xylit-Kristallsaat derjenige ist, zu dem eine Anzahl von Partikeln der Xylitkristalle, die durch ein Sichtfenster des ersten Kristallisationskessels beobachtet wird, kleiner als 50 ist, und der bestimmte Zeitraum zum Aufrechterhalten der Systemtemperatur am Ende der Kristallisation 0.5-2.0 h beträgt;
Vorgang 3, unter Verwendung der ersten Zentrifuge eine Trennbehandlung an der in Vorgang 2 erhaltenen Xylit-Zuckerpaste durchzuführen, um kristallines Xylit und eine Primärmutterlauge zu erhalten, die Primärmutterlauge über eine Rohrleitung in den Speicherbehälter für Primärmutterlauge zur zwischenzeitlichen Speicherung einzuleiten, und das kristalline Xylit durch den Heißlufttrocknungsbehälter zur Trocknungsbehandlung und den ersten Wirbelschichttrockner zur Kaltluft-Trocknungsbehandlung zu leiten, um die hergestellten Xylitkristalle zu erhalten; die Primärmutterlauge der Reihe nach durch den zweiten Verdampfer zur Konzentrationsbehandlung, den zweiten Kristallisationskessel zur Kristallisationsbehandlung und die zweite Zentrifuge zur Zentrifugationsbehandlung zu leiten, um zweikristallinen Zucker und eine Sekundärmutterlauge zu erhalten, und die Sekundärmutterlauge über eine Rohrleitung in den Speicherbehälter für Sekundärmutterlauge zur zwischenzeitlichen Speicherung einzuleiten; den zweikristallinen Zucker durch den zweiten Wirbelschichttrockner zur Trocknungsbehandlung und den Auflösebehälter für zweikristallinen Zucker zur Auflösebehandlung zu leiten, das gereinigte Wasser zuzuführen, um den zweikristallinen Zucker zu lösen und eine Zweikristallzuckerlösung zu erhalten, und die Zweikristallzuckerlösung über eine Rohrleitung in den Mischbehälter einzuleiten, um sie mit dem Rohmaterial der Xylit-Hydrierungslösung zu vermischen; und
Vorgang 4, die in Vorgang 3 erhaltene Sekundärmutterlauge der Reihe nach durch den dritten Verdampfer zur Konzentrationsbehandlung, den dritten Kristallisationskessel zur Kristallisationsbehandlung und die dritte Zentrifuge zur Zentrifugationsbehandlung zu leiten, um dreikristallinen Zucker und eine Tertiärmutterlauge zu erhalten, den dreikristallinen Zucker durch den dritten Wirbelschichttrockner zur Trocknungsbehandlung und den Auflösebehälter für dreikristallinen Zucker zur Auflösebehandlung zu leiten, das gereinigte Wasser zuzuführen, um den dreikristallinen Zucker zu lösen und eine Dreikristallzuckerlösung zu erhalten, und die Dreikristallzuckerlösung über eine Rohrleitung in den Speicherbehälter für Primärmutterlauge einzuleiten, um die Dreikristallzuckerlösung mit der Primärmutterlauge zur Wiederverwendung zu vermischen, wobei die Tertiärmutterlauge als flüssiges Xylit gespeichert wird.

4. Herstellungsverfahren nach Anspruch 3, wobei in Vorgang 1 eine Trenngrenze einer Nanofiltrationsmembran des Nanofiltrationssystems im Bereich von 300-800 Da liegt.

5. Herstellungssystem nach Anspruch 1 und Herstellungsverfahren nach Anspruch 3, wobei in Vorgang 2 der erste Kristallisationskessel ein Vakuum-Verdampfungskristallisationskessel ist, eine Rührdrehzahl bei dem frequenzvariablen Rühren schrittweise von 90 rpm auf 10 rpm verringert wird, eine Größe des Sichtfensters des ersten Kristallisationskessels 1 cm² beträgt, die Anzahl von Partikeln der Xylitkristalle durch das Sichtfenster unter Verwendung eines Mikroskops mit 10 × 10 -facher Vergrößerung beobachtet wird und das Mikroskop dazu ausgebildet ist, eine Anzahl von Partikeln der Xylitkristalle durch das Sichtfenster zu beobachten, eine Menge der Xylit-Kristallsaat 0.0004-0.0008% einer Masse einer Lösung in dem ersten Kristallisationskessel ausmacht und eine Partikelgröße der Xylit-Kristallsaat im Bereich von 80-100 mesh liegt.

6. Herstellungsverfahren nach Anspruch 3, wobei in Vorgang 2 die Xylit-Zuckerpaste nach der Durchführung der Trennbehandlung an der Xylit-Zuckerpaste unter Verwendung der ersten Zentrifuge für 5-10 s unter Verwendung einer Ethanollösung mit einer Konzentration von 80-100% besprüht und gewaschen wird, um so viel Wasser wie möglich aus dem kristallinen Xylit zu entfernen.

7. Herstellungsverfahren nach Anspruch 3, wobei in Vorgang 3 eine Temperatur einer Heißluft des Heißlufttrocknungsbehälters im Bereich von 75-85°C liegt.

## Revendications

1. Système de préparation de cristaux de xylitol, comprenant :
une cuve de mélange, une cuve de décoloration, une colonne d'échange ionique, un système de nanofiltration, un premier évaporateur, une première cuve de cristallisation, une première centrifugeuse, une cuve de séchage à air chaud et un premier sécheur à lit fluidisé, qui sont connectés séquentiellement par des conduites ;
un réservoir de stockage de liqueur mère primaire, un deuxième évaporateur, une deuxième cuve de cristallisation, une deuxième centrifugeuse, un deuxième sécheur à lit fluidisé et une cuve de dissolution de sucre dicristallin, qui sont connectés séquentiellement par des conduites ; et
un réservoir de stockage de liqueur mère secondaire, un troisième évaporateur, une troisième cuve de cristallisation, une troisième centrifugeuse, un troisième sécheur à lit fluidisé et une cuve de dissolution de sucre tricristallin, qui sont connectés séquentiellement par des conduites, dans lequel
une sortie solide de la première centrifugeuse est reliée à une entrée de la cuve de séchage à air chaud par une conduite, une sortie liquide de la première centrifugeuse est reliée à une entrée du réservoir de stockage de liqueur mère primaire par une conduite, une sortie solide de la deuxième centrifugeuse est reliée à une entrée du deuxième sécheur à lit fluidisé par une conduite, une sortie liquide de la deuxième centrifugeuse est reliée à une entrée du réservoir de stockage de liqueur mère secondaire par une conduite, la cuve de dissolution de sucre dicristallin est pourvue d'une entrée d'eau pour de l'eau purifiée, une sortie de la cuve de dissolution de sucre dicristallin est reliée à une entrée de la cuve de mélange, la cuve de dissolution de sucre tricristallin est pourvue d'une entrée d'eau pour de l'eau purifiée, une sortie de la cuve de dissolution de sucre tricristallin est reliée à l'entrée du réservoir de stockage de liqueur mère primaire par une conduite, la cuve de mélange est pourvue d'une conduite d'alimentation pour une solution d'hydrogénation, une matière première de solution d'hydrogénation de xylitol destinée à préparer les cristaux de xylitol est stockée dans la cuve de mélange, la cuve de mélange est configurée pour mélanger la matière première de solution d'hydrogénation de xylitol avec une solution de sucre dicristallin délivrée depuis la cuve de dissolution de sucre dicristallin, et une sortie du premier sécheur à lit fluidisé délivre des cristaux de xylitol préparés.

2. Système de préparation selon la revendication 1, comprenant en outre un réservoir de stockage de xylitol liquide, dans lequel une sortie de la troisième centrifugeuse est reliée à une entrée du réservoir de stockage de xylitol liquide par une conduite.

3. Procédé de préparation de cristaux de xylitol, mis en œuvre en utilisant le système de préparation de cristaux de xylitol selon la revendication 1 ou la revendication 2, le procédé comprenant :
opération 1, dans la cuve de mélange, mélanger la matière première de solution d'hydrogénation de xylitol avec la solution de sucre dicristallin selon un certain rapport, puis faire passer séquentiellement ladite matière première de solution d'hydrogénation de xylitol mélangée avec la solution de sucre dicristallin à travers la cuve de décoloration pour un traitement de décoloration, la colonne d'échange ionique pour un traitement d'élimination d'impuretés, le système de nanofiltration pour un traitement de filtration et le premier évaporateur pour un traitement d'évaporation et de concentration, afin d'obtenir un concentré de xylitol, dans lequel un indice de réfraction du concentré de xylitol est dans une plage de 78-82%, une température du concentré de xylitol est dans une plage de 90-100°C et une conductivité du concentré de xylitol est inférieure à 20 µs/cm ;
opération 2, introduire le concentré de xylitol dans la première cuve de cristallisation par une conduite, comprenant: alimenter la première cuve de cristallisation avec un volume initial de concentré de xylitol, contrôler un degré de vide de la première cuve de cristallisation à -0.095 MPa et une température de la première cuve de cristallisation à 65°C, et ajouter une quantité appropriée de germes cristallins de xylitol pour stimuler la cristallisation jusqu'à ce que des cristaux de xylitol commencent à cristalliser dans la première cuve de cristallisation, ajouter un volume additionnel de concentré de xylitol dans la première cuve de cristallisation, agiter pendant 7-12 h, et, à une fin de cristallisation, alimenter en vapeur afin d'augmenter une température du système à 66°C-68°C et maintenir la température du système pendant une certaine période de temps afin d'obtenir une pâte sucrée de xylitol, dans lequel un rapport du volume initial au volume additionnel est dans une plage de 1:0.8-1.5, l'agitation est une agitation à fréquence variable, un moment d'ajout des germes cristallins de xylitol est un moment où un nombre de particules des cristaux de xylitol observé à travers un hublot d'observation de la première cuve de cristallisation est inférieur à 50, et ladite certaine période de temps de maintien de la température du système à la fin de cristallisation est de 0.5-2.0 h ;
opération 3, effectuer, à l'aide de la première centrifugeuse, un traitement de séparation sur la pâte sucrée de xylitol obtenue à l'opération 2 afin d'obtenir du xylitol cristallin et une liqueur mère primaire, introduire la liqueur mère primaire dans le réservoir de stockage de liqueur mère primaire par une conduite pour un stockage temporaire, et faire passer le xylitol cristallin à travers la cuve de séchage à air chaud pour un traitement de séchage et le premier sécheur à lit fluidisé pour un traitement de séchage à air froid afin d'obtenir les cristaux de xylitol préparés ; faire passer séquentiellement la liqueur mère primaire à travers le deuxième évaporateur pour un traitement de concentration, la deuxième cuve de cristallisation pour un traitement de cristallisation et la deuxième centrifugeuse pour un traitement de centrifugation afin d'obtenir du sucre dicristallin et une liqueur mère secondaire, et introduire la liqueur mère secondaire dans le réservoir de stockage de liqueur mère secondaire par une conduite pour un stockage temporaire ; faire passer le sucre dicristallin à travers le deuxième sécheur à lit fluidisé pour un traitement de séchage et la cuve de dissolution de sucre dicristallin pour un traitement de dissolution, introduire l'eau purifiée pour dissoudre le sucre dicristallin afin d'obtenir une solution de sucre dicristallin, et introduire la solution de sucre dicristallin dans la cuve de mélange par une conduite pour la mélanger avec la matière première de solution d'hydrogénation de xylitol ; et
opération 4, faire passer séquentiellement la liqueur mère secondaire obtenue à l'opération 3 à travers le troisième évaporateur pour un traitement de concentration, la troisième cuve de cristallisation pour un traitement de cristallisation et la troisième centrifugeuse pour un traitement de centrifugation afin d'obtenir du sucre tricristallin et une liqueur mère tertiaire, faire passer le sucre tricristallin à travers le troisième sécheur à lit fluidisé pour un traitement de séchage et la cuve de dissolution de sucre tricristallin pour un traitement de dissolution, introduire l'eau purifiée pour dissoudre le sucre tricristallin afin d'obtenir une solution de sucre tricristallin, et introduire la solution de sucre tricristallin dans le réservoir de stockage de liqueur mère primaire par une conduite afin de mélanger la solution de sucre tricristallin avec la liqueur mère primaire pour réutilisation, la liqueur mère tertiaire étant stockée en tant que xylitol liquide.

4. Procédé de préparation selon la revendication 3, dans lequel, dans l'opération 1, un seuil de coupure d'une membrane de nanofiltration du système de nanofiltration est dans une plage de 300-800 Da.

5. Système de préparation selon la revendication 1 et procédé de préparation selon la revendication 3, dans lesquels, dans l'opération 2, la première cuve de cristallisation est une cuve de cristallisation par évaporation sous vide, une vitesse d'agitation dans l'agitation à fréquence variable diminue progressivement de 90 rpm à 10 rpm, une taille du hublot d'observation de la première cuve de cristallisation est de 1 cm², le nombre de particules des cristaux de xylitol est observé à travers le hublot d'observation au moyen d'un microscope à grossissement 10 × 10, et le microscope est configuré pour observer un nombre de particules des cristaux de xylitol à travers le hublot d'observation, une quantité des germes cristallins de xylitol représente 0.0004-0.0008% d'une masse d'une solution dans la première cuve de cristallisation, et une taille de particules des germes cristallins de xylitol est dans une plage de 80-100 mesh.

6. Procédé de préparation selon la revendication 3, dans lequel, dans l'opération 2, la pâte sucrée de xylitol est pulvérisée et lavée pendant 5-10 s à l'aide d'une solution d'éthanol d'une concentration de 80-100% après que le traitement de séparation a été effectué sur la pâte sucrée de xylitol en utilisant la première centrifugeuse, de sorte à éliminer autant d'eau que possible du xylitol cristallin.

7. Procédé de préparation selon la revendication 3, dans lequel, dans l'opération 3, une température d'un air chaud de la cuve de séchage à air chaud est dans une plage de 75-85°C.
